# EUROPÄISCHE PATENTSCHRIFT

(11) **EP 1 321 165 B1**
(45) Veröffentlichungstag und Bekanntmachung des Hinweises auf die Patenterteilung: **23.03.2005**
(21) Anmeldenummer: 02026876.9
(22) Anmeldetag: 02.12.2002
(51) Int. Cl.: A61N 1/05

(54) **Epikardelelektrodenleitung, Einführkatheter für eine solche und Elektrodenimplantationsbesteck**
Epicardial electrode lead, insertion catheter for the same and electrode implantation device
Electrode épicardique, cathéter et dispositif d'implantation correspondants

(30) Priorität: 19.12.2001 DE 10162508
(43) Veröffentlichungstag der Anmeldung: 25.06.2003
(73) Patentinhaber: BIOTRONIK Mess- und Therapiegeräte GmbH & Co Ingenieurbüro Berlin, 12359 Berlin (DE)
(72) Erfinder: Flach, Erhard, 12305 Berlin (DE)
(74) Vertreter: Heinze, Ekkehard, Dipl.-Phys. Dr.

(56) Entgegenhaltungen:
- US-A- 4 799 499
- US-A- 5 443 492
- US-A- 5 571 162
- US-A- 5 693 081
- US-A1- 2001 020 179

## Beschreibung

Die Erfindung betrifft eine Epikardelektrodenleitung (vereinfacht auch bezeichnet als "epikardiale Elektrode") gemäß dem Oberbegriff des Anspruchs 1 sowie Hilfsmittel zur Implantation und Fixierung einer solchen am Epikard in einem minimalinvasiven Eingriff.

Implantierbare Elektrodenleitungen zum Einsatz im oder am Herzen sind in Verbindung mit implantierbaren Herzschrittmachern entwickelt worden und seit langem in großer Vielgestaltigkeit bekannt. Bei weitem die größte Bedeutung haben hierbei intrakardial verlegte Elektrodenleitungen erlangt, die über einen transvenösen Zugang direkt in das Herz geführt werden. Für diese Elektroden sind verschiedene Arten der Fixierung an der Innenwand des Herzens bzw. im Trabekelwerk der Herzkammer vorgeschlagen und auch praktisch realisiert worden.

Hierunter befinden sich auch verschiedene Arten von "Einschraubelektroden", die - bevorzugt am distalen Ende - eine Einschraubwendel zur aktiven Fixierung tragen. Daneben gibt es auch intrakardiale Elektroden mit Widerhaken- bzw. Finnenanordnung zur atraumatischen Fixierung im Trabekelwerk. Desweiteren sind auf spezielle Weise gekrümmte und/oder verzweigte Elektrodenleitungen bekannt, bei denen die vorgeprägte Grundform ein zuverlässiges Anliegen an der Herzwand und damit die sichere Übertragung von Stimulationsimpulsen des Schrittmachers an diese gewährleisten soll.

Es sind auch aufwendige Einschraubelektrodenkonstruktionen bekanntgeworden, bei denen eine Einschraubwendel senkrecht zur Längserstreckung der Elektrodenleitung verläuft und folglich auch bei einer im wesentlichen zur Herzwandung parallelen Lage der Elektrodenleitung praktisch senkrecht in die Herzwandung eindringen kann. Es versteht sich, daß eine solche Konstruktion aufgrund der erforderlichen Umlenkung der Antriebskraft aufwendig und relativ voluminös ist. Letzteres macht sie für den Einsatz bei einem minimalinvasiven Eingriff praktisch ungeeignet.

Während für den dauerhaften Einsatz zur Impulsübertragung von fest implantierten Schrittmachern im wesentlichen nur intrakardiale Elektroden eingesetzt werden, kommen epikardiale Elektroden vor allem zur temporären Stimulation des Herzens bei oder nach operativen Eingriffen zum Einsatz. Desweiteren werden sie in Form großflächiger Flächenelektroden (Patch-Elektroden) in Verbindung mit implantierbaren Defibrillatoren eingesetzt - im letzteren Einsatzgebiet haben sie sich aber wegen des enormen Operationsaufwandes am offenen Herzen nur noch für besondere Konstellationen behaupten können.

Zunehmend Bedeutung gewinnen epikardiale Elektroden für die Übertragung von Stimulationsimpulsen zum linken Ventrikel. Der linke Ventrikel ist über den transvenösen Zugang nur umständlich und schwer zu erreichen, denn der "klassische" transvenöse Weg führt über die Superior Vena Cava in das rechte Atrium, aus diesem heraus in den Koronarsinus und von diesem - annähernd in einem rechten Winkel abzweigend - in eines der linksseitigen venösen Koronargefäße. Die Führung der intrakardialen Elektrode durch die vielen Biegungen und teilweise engen Passagen erfordert außerordentliches Geschick und große Erfahrung des Operateurs und gelingt auch dann nicht immer.

Für diesen speziellen Einsatzort linkes Ventrikel ist der epikardiale Zugang im Rahmen eines minimalinvasiven Eingriffs grundsätzlich wesentlich leichter ausführbar. Es kommt hierbei darauf an, die Elektrode in den Zwischenraum zwischen dem Herzbeutel (Perikard) und der Außenwand des Myokards (Epikard) zu führen, ohne daß dabei Blut mit in den Herzbeutel hinein gelangt. Dieses würde innerhalb des Perikard zu einer sogenannten Tamponade - einem großflächigen Gerinsel - führen, welches Funktion und Stoffwechsel des darunterliegenden Myokards empfindlich stören kann. Sobald mit einer geeigneten Operationstechnik und hinreichender Erfahrung dieses mit der Punktion des Perikards verbundene Problem gemeistert werden kann, kann die Verlegung einer epikardialen Elektrode am linken Ventrikel unter Umständen der intrakardialen Implantation vorzuziehen sein.

Es ist bekannt, epikardiale Elektroden auf minimalinvasive Weise durch die Haut und das darunterliegende Gewebe des Patienten und das Perikard hindurch vorzunehmen, wobei die Elektrode bzw. Elektrodenleitung selbsttätig eine zum Myokard parallele bzw. "tangentiale" Lage einnimmt. Bei dieser Lage können die für den intrakardialen Einsatz bekannten Einschraubelektroden ebensowenig verwendet werden wie andere bekannte Fixierungsmechanismen intrakardialer Elektrodenleitungen.

Es sind daher epikardiale Elektroden mit senkrecht zur Längserstreckung verlaufenden Schrauben oder Haken vorgeschlagen und bis zu einem gewissen Grade auch praktisch eingesetzt worden. Außerdem ist ein Verfahren bekanntgeworden, bei dem eine Elektrode mit axial verlaufender distaler Fixierungsschraube mittels eines geeigneten Einführbesteckes in einem spitzen Winkel von etwa 30° in das Myokard eingeschraubt wird. Es ist leicht vorstellbar, daß eine solche Elektrode den anatomischen Gegebenheiten am Myokard nur unzureichend gerecht wird und zwischen Myokard und Elektrode dauerhafte und erhebliche Biegespannungen und punktuelle Druckspitzen auftreten, die weder der Elektrodenfunktion noch der Haltbarkeit des Implantats zum Vorteil gereichen.

Der Erfindung liegt daher die Aufgabe der Bereitstellung einer verbesserten Epikardelektrodenleitungen zugrunde, die eine anatomisch korrekte und somit sichere Fixierung ermöglicht. Desweiteren soll ein geeignetes Implantationsbesteck für diese Elektrodenleitung bereitgestellt werden.

Diese Aufgabe wird hinsichtlich der eigentlichen Elektrode durch eine Epikardelektrodenleitung mit den Merkmalen des Anspruchs 1 und hinsichtlich geeigneter Hilfsmittel durch ein Einführkatheter gemäß Anspruch 5 bzw. ein Elektrodenimplanta tionsbesteck gemäß Anspruch 11 gelöst.

Die Erfindung schließt den grundlegenden Gedanken ein, am distalen Ende - dem Elektrodenkopf - der Elektrodenleitung ein tangential wirkendes Eingriffselement vorzusehen, welches bei parallel zur Epikardoberfläche orientierter Leitung in das Epikard hineingedreht werden kann. Sie schließt weiter den Gedanken ein, dieses Eingriffselement als einen Fixierungshakenabschnitt auszubilden.

In einer nicht zur Erfindung gehörenden Ausführung ist der Elektrodenkopf mindestens abschnittsweise mit geringem Abstand von einer Drahtwendel umgeben, deren Ende aufgeweitet und/oder im wesentlichen gestreckt ausgeführt ist und den Fixierungshakenabschnitt bildet. Der Elektrodenkopf hat also - mit anderen Worten - eine distale und axial ausgerichtete Einschraubwendel, bei der zumindest der letzte "Gang" eine gegenüber dem Elektrodenkopf größere laterale Erstreckung (bei einem im Querschnitt kreisförmigen Elektrodenkopf einen größeren Durchmesser) hat. Das freie Ende der Wendel ragt also radial über die Wandung des Elektrodenkopfes hinaus und ist im Bezug auf diese tangential orientiert.

Dieser Aufbau ist von bestechender Einfachheit und erfordert nahezu keine Vergrößerung des Elektrodenkopfes, was ihn für minimalinvasive Eingriffe besonders geeignet macht. Von Bedeutung sind auch die durch den einfachen Aufbau bedingten niedrigen Gestehungskosten.

Zur Implantation wird die Elektrode in den Zwischenraum zwischen Perikard und Epikard geführt, an das Epikard angelegt und mittels eines geeignet ausgebildeten Führungsdrahtes die Drahtwendel derart gedreht, daß das freie Ende (der Fixierungshakenabschnitt) in das Epikard einsticht bzw. einhakt. Durch Fortsetzung der Schraubbewegung werden dann auch nachfolgende "Gänge" in das Epikard eingedreht und hierbei sukzessive elastisch aufgebogen bzw. aufgeweitet, wodurch letztlich mehrere Eingriffsabschnitte zwischen Wendel und Epikard geschaffen werden. Der Elektrodenkopf wird hierdurch sicher am Epikard gehalten.

In einer hierzu alternativen Ausführung auf Anspruch 1 ist am Elektrodenkopf eine Drahtspirale angebracht, deren äußeres Ende den Fixierungshakenabschnitt bildet. Auch eine Elektrode mit diesem Aufbau ist kostengünstig und leicht zu implantieren.

Das Fixierungsverfahren ähnelt dem oben beschriebenen - mit dem Unterschied, daß die erwähnte Spirale nur einen Eingriffsabschnitt mit dem Epikard hat und das Fixieren nur den Bruchteil einer Umdrehung der Spirale bzw. des Elektrodenkopfes erfordert. Die Festigkeit der Verankerung kann natürlich diejenige bei der oben erwähnten, annähernd zylindrischen Schraubwendel nicht erreichen.

Zur Erleichterung der Fixierung ist der Fixierungshakenabschnitt - also im speziellen Fall die erwähnte Drahtwendel oder -spirale - federelastisch ausgeführt und hat ein angespitztes oder mit einer Schneide versehenes Ende. Dies ermöglicht ein Eindringen in das Epikard mit sehr geringem Kraftaufwand des Operateurs.

Der als wesentliche Komponente des o. a. Implantationsbestecks vorgeschlagene Einführkatheter sichert in vorteilhafter Weise, daß der Fixierungshakenabschnitt der Elektrode im Zuge des Eindrehens in das Myokard sich nicht versehentlich auch in das Perikard eindreht. Die Elektrode muß nämlich gegenüber dem Perikard frei beweglich sein. Das vorgeschlagene Einführkatheter ist für beide oben erwähnten Alternativen der Ausführung des Fixierungshakenabschnittes (als Wendel- oder Spiralabschnitt) grundsätzlich gleichermaßen geeignet.

Das distale Ende des Katheters kann bis zur Stirnfläche hin teilweise geöffnet sein - was aus derzeitiger Sicht die bevorzugte Ausführung darstellt. Insbesondere hat der Kunststoffkörper eine im wesentlichen zylindrische, insbesondere kreiszylindrische, Außenform und ein im wesentlichen zylinderabschnittförmiges, insbesondere halbzylindrisches, distales Ende.

Der Kunststoffkörper des Einführkatheters ist bevorzugt in seinem distalen Endabschnitt steifer als im sich daran anschließenden Schaftabschnitt ausgeführt, denn der letztere muß für die (weiter unten genauer beschriebene) Einführung eine gewisse Flexibilität haben. Zur erleichterten Einführung trägt im übrigen eine abgeschrägte oder abgerundete distale Stirnfläche des Katheters bei, und das Vorschieben der Elektrodenleitung im gesetzten Einführkatheter erleichtert eine Gleitbeschichtung im Lumen. Bevorzugt ist der Einführkatheter in seinem distalen Endbereich mit einer an die Herzkontur angepaßten Längskrümmung versehen, um ein möglichst "glattes" Anlegen der Elektrodenleitung an das Myokard zu erreichen und damit eine leichte und sichere Fixierung zu ermöglichen.

Zur Ausführung der Fixierung, d. h. zum Hineindrehen des Fixierungshakenabschnittes in das Myokard, ist als Bestandteil des Implantationsbestecks ein spezieller Führungsdraht (Mandrin) vorgesehen, der mit dem Eingriffshakenabschnitt - bzw. dem gesamten Elektrodenkopf, sofern der Eingriffshakenabschnitt an diesem drehfest angebracht ist - in drehfesten Eingriff bringbar. An seinem proximalen Ende befindet sich ein Betätigungsabschnitt zur Drehmomentübertragung auf den Eingriffshakenabschnitt (bzw. Elektrodenkopf) und zur definierten Bestimmung von dessen Winkellage. In einer besonders einfachen Ausführung handelt es sich hierbei um einen Schraubendreher-Eingriffsabschnitt gemäß einem der gängigen Standards (Schlitz, Kreuzschlitz oder Innensechskant o. ä.).

Vorteile und Zweckmäßigkeiten der Erfindung ergeben sich im übrigen aus den Unteransprüchen sowie der nachfolgenden Beschreibung bevorzugter Ausführungsbeispiele anhand der Figuren. Von diesen zeigen:
- Fig. 1: eine perspektivische Darstellung des Endes einer Epikardelektrodenleitung gemäß einer ersten Ausführungsform,
- Fig. 2A und 2B: zwei Ansichten des distalen Endes eines zur Elektrodenleitung nach Fig. 1 gehörigen Einführkatheters und
- Fig. 3: eine Skizze des distalen Endes einer Epikardelektrodenleitung gemäß einer weiteren Ausführungsform.

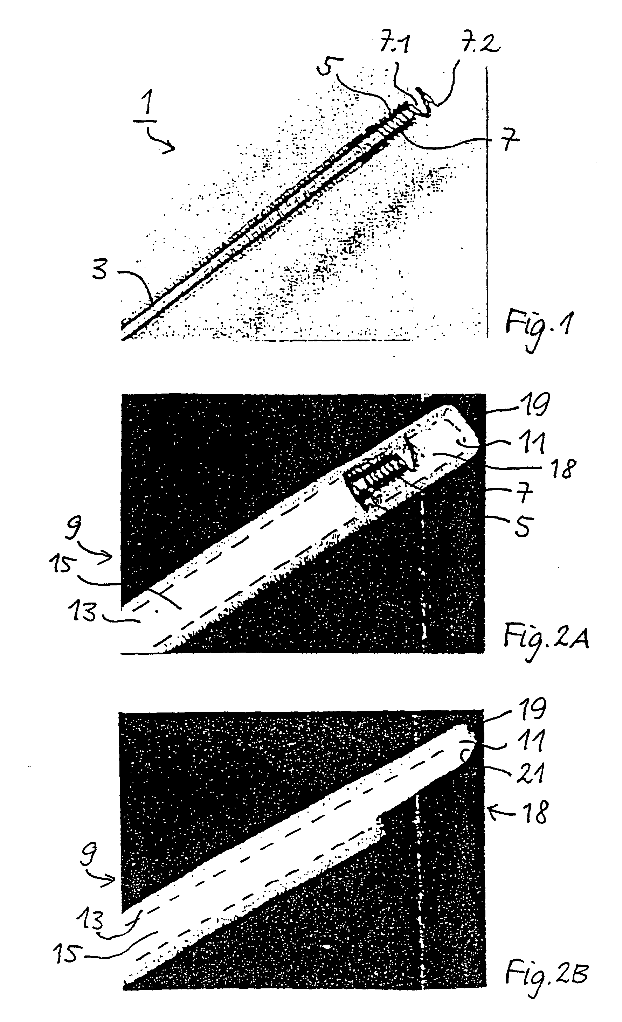

Fig. 1 zeigt den distalen Endabschnitt einer im wesentlichen zylindrischen Elektrodenleitung 1, die eine Zuleitung 3 und einen Elektrodenkopf 5 umfaßt. Der Elektrodenkopf 5 ist über den größten Teil seiner Länge von einer federelastisch ausgeführten zylindrischen Schraubwendel 7 umgeben, deren letzter Gang 7.1 in Längsrichtung etwas gestreckt und über den maximalen Durchmesser des Elektrodenkopfes 5 aufgeweitet ist. Das distale Ende 7.2 der Schraubwendel ist angespitzt.

In Fig. 2A und 2B ist in zwei Ansichten ein Einführkatheter 9 aus Kunststoff mit einer harten bzw. steifen Spitze 11 und einem mittelharten bzw. halbsteifen Schaft 13 dargestellt. Der Einführkatheter 9 ist im wesentlichen über seine gesamte Länge zylindrisch und hat ein mit einer Gleitbeschichtung versehenes Lumen 15, dessen Durchmesser auf den (maximalen) Außendurchmesser der Elektrodenleitung 1, d. h. den Durchmesser des letzten Ganges 7.1 der Schraubwendel 7, abgestimmt ist. In Fig. 2A ist der Einführkatheter 9 mit eingeschobener Elektrodenleitung 1 gezeigt, wobei der Elektrodenkopf 5 mit der Schraubwendel 7 zu erkennen ist. Dieser liegt nämlich im distalen Endbereich 17 des Einführkatheters 9, der halbzylindrisch ausgeführt ist, also eine langgestreckte seitliche Öffnung 18 hat, die bis zur distalen Stirnfläche 19 reicht. Dort ist eine Abschrägung 21 zur Erleichterung der Einführung des Katheters bei dem weiter unten beschriebenen minimalinvasiven Eingriff vorgesehen.

Zur Implantation der Elektrodenleitung 1 nach Fig. 1 unter Zuhilfenahme des Einführkatheters 9 nach Fig. 2A und 2B wird folgende Zugangsmethode vorgeschlagen:
1. Lokale Anästhesie unterhalb Sternum und Punktion des Perikards.
2. Röntgendarstellung des Herzens durch Injektion von Kontrastmitteln in den Herzbeutel.
3. Sondieren des Herzbeutels mit einem Seldinger-Draht.
4. Einführen einer Schleuse mit Hilfe eines Dilatators.
5. Einführung eines steuerbaren Ablationskatheters zur Lagesteuerung der Peel Away Schleuse zur temporären Stimulation.
6. Darstellung der Koronaraterien über eine intravaskulären Zugang zur Vermeidung von Gefäßverletzungen durch mögliche aktive Elektrodenfixierungen.
7. Nach Positionierung des Endes des Führungskatheters Austausch des steuerbaren Ablationskatheters durch permanent implantierbare Elektrode.

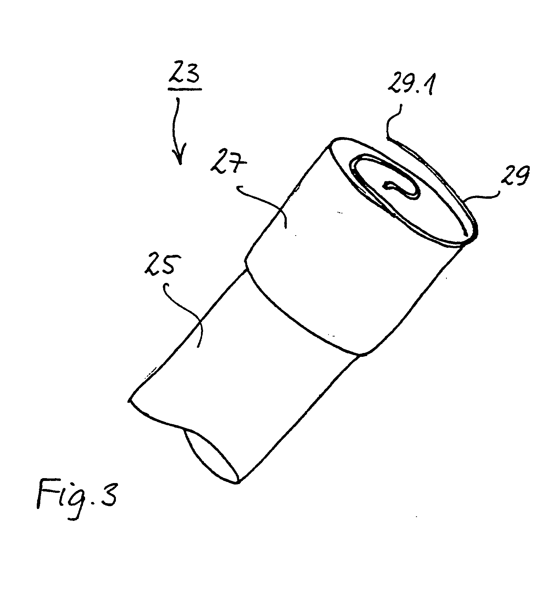

Fig. 3 zeigt in Art einer Prinzipskizze eine modifizierte Elektrodenleitung 23 mit einer Zuleitung 25 und einem Elektrodenkopf 27, die denen der Elektrodenleitung 1 nach Fig. 1 ähnlich sind. Der wesentliche Unterschied besteht darin, daß bei der vorliegenden Ausführung anstelle einer um den Umfang des Elektrodenkopfes gewickelten Schraubwendel eine am distalen Ende in den Elektrodenkopf eingesetzte Drahtspirale 29 vorgesehen ist, deren äußeres Ende über den Umfang des Elektrodenkopfes 27 radial hinausreicht und im wesentlichen tangential verläuft. Auch die Drahtspirale 29 hat ein angespitztes Ende 29.1. Zur Implantation kann im wesentlichen die oben skizzierte Zugangsmethode angewandt werden.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

### Bezugszeichenliste

- 1; 23: Elektrodenleitung
- 3; 25: Zuleitung
- 5; 27: Elektrodenkopf
- 7: Schraubwendel
- 7.1: letzter Gang
- 7.2; 29.1: angespitztes Ende
- 9: Einführkatheter
- 11: Spitze
- 13: Schaft
- 15: Lumen
- 17: distaler Endbereich
- 18: seitliche Öffnung
- 19: distale Stirnfläche
- 21: Abschrägung
- 29: Drahtspirale

## Patentansprüche

1. Epikardelektrodenleitung (1; 23) zur minimalinvasiven Implantation und Verankerung am Epikard, mit einer langgestreckten Zuleitung (3; 25) und einem distal an dieser angeordneten und im wesentlichen mit ihr konzentrischen Elektrodenkopf (5; 27),
wobei
der Eletrodenkopf einen über seine Außenfläche vorstehenden und im wesentlichen tangential unter einem spitzen Winkel zu dieser verlaufenden Fixierungshakenabschnitt (7.1; 29) aufweist derart, dass am Elektrodenkopf (27) eine Drahtspirale (29) angebracht ist, deren äußeres Ende den Fixierungshakenabschnitt bildet.

2. Epikardelektrodenleitung nach Anspruch 1
**dadurch gekennzeichnet, daß**
der Elektrodenkopf (5; 27) im wesentlichen zylindrisch, sphärisch oder kegelstumpfförmig ausgebildet und der Fixierungshakenabschnitt (7.1; 29) im wesentlichen tangential zur Oberfläche des Elektrodenkopfes verläuft.

3. Epikardelektrodenleitung nach einem der vorangehenden Ansprüche,
**dadurch gekennzeichnet, daß**
der Fixierungshakenabschnitt (7.1; 29), insbesondere die gesamte Drahtspirale, federelastisch ausgeführt ist.

4. Epikardelektrodenleitung nach einem der vorangehenden Ansprüche,
**dadurch gekennzeichnet, daß**
der Fixierungshakenabschnitt (7.1; 29) ein distales Ende mit einer Spitze oder Schneide (7.2; 29.1) aufweist.

5. Epikardelektrodenleitung (1; 23) nach einem der vorangehenden Ansprüche, mit Einführkatheter (9)
mit einem langgestreckten, an die Form und Ausmessungen der Epikardelektrodenleitung angepaßten Kunststoffkörper (11, 13), dessen distales Ende seitlich offen (18) ist derart, daß der Fixierungshakenabschnitt (7.1; 29) der Epikardelektrodenleitung durch Drehen derselben innerhalb des Einführkatheters in Eingriff mit einem an der Öffnung anliegenden Epikardgewebeabschnitt gebracht werden kann.

6. Epikardelektrodenleitung nach Anspruch 5,
**dadurch gekennzeichnet, daß**
der Kunststoffkörper (11, 13) eine im wesentlichen zylindrische, insbesondere kreiszylindrische, Außenform und ein im wesentlichen zylinderabschnittförmiges, insbesondere halbzylindrisches, distales Ende (17) hat.

7. Epikardelektrodenleitung nach Anspruch 5 oder 6, **gekennzeichnet durch**
einen steifen distalen Endabschnitt (11), an den sich proximal ein halbsteifer Schaftabschnitt (13) anschließt.

8. Epikardelektrodenleitung nach einem der Ansprüche 5 bis 7, **gekennzeichnet durch**
eine abgeschrägte (21) oder abgerundete distale Stirnfläche (19).

9. Epikardelektrodenleitung nach einem der Ansprüche 5 bis 8, **gekennzeichnet durch**
eine Gleitbeschichtung eines zur Aufnahme der Epikardelektrodenleitung vorgesehenen Lumens.

10. Epikardelektrodenleitung nach einem der Ansprüche 5 bis 9, **gekennzeichnet durch**
eine an die Oberflächenkontur des Herzens angepaßte Krümmung im Längsverlauf des distalen Endabschnittes.

11. Elektrodenimplantationsbesteck mit einer Epikardelektrodenleitung (1; 23) nach einem der Ansprüche 1 bis 4 und
einem Einführkatheter (9) nach einem der Ansprüche 5 bis 10.

12. Elektrodenimplantationsbesteck nach Anspruch 11, mit einem drehfest mit dem Elektrodenkopf der Epikardelektrodenleitung koppelbaren Führungsdraht, an dessen proximalem Ende ein Betätigungsabschnitt zur Drehmomentübertragung auf den Elektrodenkopf und zur definierten Bestimmung von dessen Winkellage vorgesehen ist.

13. Elektrodenimplantationsbesteck nach Anspruch 12,
**dadurch gekennzeichnet, daß**
der Betätigungsabschnitt als genormter Schraubendreher-Eingriffsabschnitt ausgeführt ist.

## Claims

1. Epicardial electrode lead (1; 23) for minimally invasive implantation and anchoring to the epicardium, with an elongated supply lead (3; 25) and an electrode head (5; 27) distally attached thereto and substantially concentric therewith,
wherein the electrode head comprises a fixation-hook section (7.1; 29) that extends beyond its outer surface and is oriented substantially tangential to the electrode head, at an acute angle, such that to the electrode head (27) a spiral wire (29) is attached, the outer end of which forms the fixation-hook section.

2. Epicardial electrode lead according to claim 1,
**characterized in that** the electrode head (5; 27) has substantially the shape of a cylinder, sphere or truncated cone and the fixation-hook section (7.1; 29) runs substantially tangential to the surface of the electrode head.

3. Epicardial electrode lead according to one of the preceding claims,
**characterized in that** the fixation-hook section (7.1; 29), in particular the entire spiral wire, has a resilient, spring-like construction.

4. Epicardial electrode lead according to one of the preceding claims,
**characterized in that** the fixation-hook section (7.1; 29) comprises a distal end with a sharp tip or cutting edge (7.2; 29.1).

5. An epicardial electrode lead (1; 23) according to one of the preceding claims, with an introducer (9), with an elongated plastic body (11, 13) matched to the shape and dimensions of the epicardial electrode lead and having a distal end that is open at the side (18) such that the fixation-hook section (7.1; 29) of the epicardial electrode lead can, by rotating the lead within the introducer, be brought into engagement with a section of epicardial tissue adjacent to the opening.

6. Epicardial electrode lead according to Claim 5,
**characterized in that** the plastic body (11, 13) has an external surface in substantially the shape of a cylinder, in particular a circular cylinder, and a distal end (17) in substantially the shape of a section of a cylinder, in particular a half-cylinder.

7. Epicardial electrode lead according to Claim 5 or 6,
**characterized by** a stiff distal end section (11), immediately proximal to which is a semi-stiff shaft section (13).

8. Epicardial electrode lead according to one of claims 5 to 7,
**characterized by** a beveled (21) or rounded distal end surface (19).

9. Epicardial electrode lead according to one of claims 5 to 8,
**characterized by** a slippery coating of a lumen provided to contain the epicardial electrode lead.

10. Epicardial electrode lead according to one of claims 5 to 9,
**characterized by** a longitudinal curvature of the distal end section that is matched to the surface contour of the heart.

11. Set of electrode implantation instruments with an epicardial electrode lead (1; 23) according to one of claims 1 to 4 and an introducer (9) according to one of claims 5 to 10.

12. Set of electrode implantation instruments according to Claim 11, with a guide wire that can be nonrotatably coupled to the electrode head of the epicardial electrode lead, at the proximal end of which is provided an actuation section for transmitting torque to the electrode head and for precisely determining its angular position.

13. Set of electrode implantation instruments according to Claim 12,
**characterized in that** the actuator section has a configuration such as is standard for the engagement end of a screwdriver.

## Revendications

1. Conduit d'électrode épicardiaque (1 ; 23) pour implantation et ancrage les moins invasifs possibles à l'épicarde, comportant une conduite d'amenée longiligne (3 ; 25) et une tête d'électrode (5 ; 27) disposée de façon distale par rapport à elle et lui étant, pour l'essentiel, concentrique,
où la tête d'électrode présente une section de crochet de fixation (7.1 ; 29) dépassant de sa surface extérieure et, pour l'essentiel, tangentielle avec un angle aigu par rapport à celle-ci, de manière telle qu'une spirale en fil (29) est montée à la tête d'électrode (27), dont l'extrémité extérieure forme la section de crochet de fixation.

2. Conduit d'électrode épicardiaque selon la revendication 1,
**caractérisé en ce que**
la tête d'électrode (5 ; 27) est, pour l'essentiel, de forme cylindrique, sphérique ou en tronc de cône et que la section de crochet de fixation (7.1 ; 29) s'étend, pour l'essentiel, de façon tangentielle à la surface de la tête d'électrode.

3. Conduit d'électrode épicardiaque selon l'une quelconque des revendications précédentes,
**caractérisé en ce que**
la section de crochet de fixation (7.1 ; 29), en particulier la totalité de la spirale en fil, est réalisée de façon à être élastique à la façon d'un ressort.

4. Conduit d'électrode épicardiaque selon l'une quelconque des revendications précédentes,
**caractérisé en ce que**
la section de crochet de fixation (7.1 ; 29) présente une extrémité distale comportant une pointe ou une arête tranchante (7.2 ; 29.1).

5. Conduit d'électrode épicardiaque (1 ; 23) selon l'une quelconque des revendications précédentes, comportant un cathéter d'introduction (9), avec un corps en matière synthétique (11, 13), allongé, adapté à la forme et aux dimensions du conduit d'électrode épicardiaque, dont l'extrémité distale est ouverte latéralement (18) de manière telle que la section de crochet de fixation (7.1 ; 29) du conduit d'électrode épicardiaque peut être engagée par rotation de celle-ci à l'intérieur du cathéter d'introduction avec une section de tissu épicardiaque figurant contre l'ouverture.

6. Conduit d'électrode épicardiaque selon la revendication 5,
**caractérisé en ce que**
le corps en matière synthétique (11, 13) présente une forme extérieure pour l'essentiel cylindrique, en particulier cylindrique circulaire, et possède une extrémité distale pour l'essentiel en forme de section de cylindre, en particulier semi-cylindrique.

7. Conduit d'électrode épicardiaque selon la revendication 5 ou 6,
**caractérisé par**
une section d'extrémité (11) rigide, distale, à laquelle se raccorde de façon proximale une section d'arbre (13) semi-rigide.

8. Conduit d'électrode épicardiaque selon l'une quelconque des revendications 5 à 7,
**caractérisé par**
une surface frontale (19) distale en biseau (21) ou arrondie.

9. Conduit d'électrode épicardiaque selon l'une quelconque des revendications 5 à 8,
**caractérisé par**
un revêtement antifriction d'un lumen prévu pour recevoir le conduit d'électrode épicardiaque.

10. Conduit d'électrode épicardiaque selon l'une quelconque des revendications 5 à 9,
**caractérisé par**
une courbure adaptée au contour de la surface du coeur dans le sens longitudinal de la section d'extrémité distale.

11. Une trousse d'implantation d'électrodes comportant un conduit d'électrode épicardiaque (1; 23) selon l'une quelconque des revendications 1 à 4 et un cathéter d'introduction (9) selon l'une quelconque des revendications 5 à 10.

12. Trousse d'implantation d'électrodes selon la revendication 11, comportant un fil de guidage pouvant être accouplé sans pouvoir pivoter à la tête d'électrode du conduit d'électrode épicardiaque, à l'extrémité proximale duquel est prévue une section d'actionnement pour la transmission de couple à la tête d'électrode et pour déterminer de façon définie sa position angulaire.

13. Trousse d'implantation d'électrodes selon la revendication 12,
**caractérisée en ce que**
la section d'actionnement est constituée en tant que section normalisée d'engagement de tournevis.
